# EUROPEAN PATENT APPLICATION

(11) **EP 4 159 747 A1**
(43) Date of publication of application: **05.04.2023**
(21) Application number: 21812248.9
(22) Date of filing: 24.05.2021
(51) Int. Cl.: C07K 14/415, C12N 1/20, A61K 38/16, A23L 33/18, A23K 20/147

(54) **COMPOSITION FOR NEUTRALIZING CORONAVIRUS**

(30) Priority: 25.05.2020 KR 20200062657
(71) Applicant: Bio3s Inc., Gwangju 61186 (KR)
(72) Inventor: KIM, Du Woon, Gwangju 61033 (KR); CHO, Jong Youn, Incheon 22736 (KR)
(74) Representative: ZBM Patents - Zea, Barlocci & Markvardsen
(86) International application number: PCT/KR2021/006464
(87) International publication number: WO 2021/241969

(57) **Abstract**

The present disclosure relates to a novel protein that specifically binds to a coronavirus, and a composition for neutralizing a coronavirus. A novel protein provided in the present disclosure is a novel protein in which virus binding ability is maintained but toxicity is eliminated on the basis of virus binding properties of bean-derived protein lectins, and thus can be used as a material for preventing the infection of a coronavirus or alleviating infectious diseases.

## Description

### Technical Field

The present disclosure relates to a novel protein that specifically binds to a coronavirus, and a composition for neutralizing a coronavirus.

### Background Art

Coronavirus infectious pneumonia, which started in Wuhan, China in 2019, is an acute infectious disease known to have infected 1.8 million or more people worldwide and killed 110,000 or more people worldwide as of April 2020. Research to suppress the infection of this virus, named Corona 19, is being conducted around the world, and the development of materials with scientific evidence and proven safety is of high importance for the salvation of mankind.

Coronavirus was first discovered in chickens in 1937, followed by animals such as dogs, pigs, and birds, and then in humans in 1965. So far, the coronavirus has been recognized as a virus that rarely infects humans and mainly infects animals such as dogs, pigs, and cattle. It is one of several viruses that cause respiratory symptoms even when infecting humans, and only caused a simple cold or intestinal disease such as diarrhea, which is not a high risk for children. However, as the causative bacteria of Severe Acute Respiratory Syndrome (SARS), which first occurred in mid-March 2003 and caused more than 100 deaths and 3,000 patients worldwide, was known to be a novel (mutant) coronavirus, it gradually started to get attention.

Coronaviruses are known to mainly cause pneumonia and enteritis in humans and animals, and are also known to occasionally cause nervous system infections and hepatitis. Coronaviruses belong to the family Coronaviridae and are positive sense RNA viruses with a spherical outer membrane of about 100 to 120 nm in size. Coronaviruses consist of a total of five structural proteins, including the outermost spike protein (S), hemagglutinin-esterase (HE) protein, transmembrane (M) protein, small membrane (E) protein, and nucleocapsid (N) protein (Lai and Homes, 2001. Fields Virology). Among the above, the spike protein acts as a ligand binding to a cell receptor and induces fusion between the host cell and the virus, and is known as the most mutable protein. Coronaviruses are divided into three major serotypes. Examples of serotype 1 includes a causative agent of developing infectious enteritis in pigs. This disease is also occurring in Korea, and is being treated as important in the domestic pig industry because it causes an incidence rate of close to 100% and mortality rate of 70% or more in young piglets. Examples of stereotype 2 include bovine coronavirus infection, which is being treated as important in livestock. Examples of stereotype 3 includes chicken infectious bronchitis, which causes respiratory, urinary and egg-laying disorders in chickens. Unlike other serotype viruses, bovine coronavirus is distinguished morphologically, serologically and genetically by having a second spike, hemagglutinin, in the external membrane of the virus. In particular, serotype 2 is very important as the serotype to which the SARS coronavirus, which occurred mainly in China in 2002 and terrorized the whole world, belongs. According to WHO statistics, it is reported that the SARS coronavirus has infected a total of 8,500 people, of which 800 have died. Recently, it was found that the SARS coronavirus originated from bats and was transmitted to humans. Accordingly, coronavirus infection due to cross-species transmission is a big issue.

Targets for coronavirus control may be divided into cell invasion inhibition, cytokine (IFN) suppression, RNA replication suppression, and viral protease inhibition. Virus cell invasion inhibition technology is studied for the purpose of suppressing viral infection at an early stage. It has been found that the protein of the Corona 19 virus adheres well to the ACE2 receptor of specific cells (AT2) among lung cells. Research is continuing to neutralize the virus by incapacitating this receptor to suppress host infection.

Canavalia ensiformis is a leguminous plant used for animal feed and human food, and is called Canavalia ensiformis because of the large and broad pods that look like the blades of a straw cutter. Canavalia ensiformis is known to have antioxidant and antibacterial activity. The leaves grow alternately and are compound leaves composed of three small leaves. Long flower stalks come out in late summer from July to August, and pale magenta or white flowers bloom in inflorescences. There are about ten seeds (beans) in the pod. The seeds (beans) are used as medicine, cooked in rice, or brewed into tea. Originally, they are native to tropical Asia, but due to climate change, they are also now cultivated in Korea.

Lectin is a generic term for carbohydrate-binding proteins that specifically bind to monosaccharides or oligosaccharides, and is a protein that neutralizes and captures viral infections by binding to glycoproteins on the surface of bacteria and viruses. Recently, attempts have been made to suppress and neutralize viruses using carbohydrate-binding agents that bind to viruses, that is, lectins. For example, there are anti-HIV lectins Cyanovirin-N and banlec, anti-IAV lectins ESA-2, anti-HCV lectins Galanthus nivalis agglutinin (GNA), and the like. The lectin suppresses infection and transmission by targeting and binding to viral envelope proteins, particularly N-linked oligosaccharides of the coronavirus spike protein, and is also a potential candidate for bactericides and therapeutic agents.

Lectins act as an intrinsic defense mechanism that ensures survival by eliciting a negative response to predators in plants. Many lectins cause inflammation, damage nerves and kill cells, while some lectins increase blood viscosity, disrupt gene expression, and disturb endocrine function. These properties of lectins are referred to as lectin toxicity in this document.

ConcanavalinA (ConA) derived from Canavalia ensiformis is a protein belonging to the lectin family that binds to sugars such as mannose or glucose at the monosaccharide binding site. Under physiological conditions, ConA is a tetramer, and selectively binds to glycoproteins of cell surface including α-mannopyranosyl and α-glucopyranosyl residues. This feature has a wide range of applications in biology and biomedicine, and is often used as a binding agent for pathogen-physiological reactions. ConA makes a feature of binding to an envelope protein virus such as Dengue virus (DENV), Hepatitis C Virus (HCV), Herpes Virus (HSV), Human Immunodeficiency Virus (HIV), Influenza A Virus (IAV), and murine RNA tumor virus. It has recently been reported to have a strong binding force with norovirus, a non-envelope protein virus. However, concanavalinA has a toxicity issue that causes T-cell division.

In order to eliminate the toxicity of concanavalinA, a part of the protein is truncated, and a mass spectrometer is used to analyze and confirm the exact amino acid sequence of the produced protein. Amino acid sequencing through protein mass measurement fragments the peptide in a mass spectrometer and calculates the difference in molecular weight between mass spectrometry spectra of each piece to analyze the amino acid sequence. This analysis method is called MS/MS or tandem MS. As the MS/MS method, CID, HCD, PQD, ETD, and ECD methods are used. One of the reasons why post-translational modification analysis of proteins using a mass spectrometer is the most powerful analysis method is that it may map amino acids in which post-translational modifications of proteins occur in proteins. The tandem mass analysis method in a mass spectrometer refers to a method of fragmenting peptide ions in a specific gas-phase. Herein, by comparing the mass difference between the fragmented signal peaks with the mass value of each amino acid or the amino acid mass value that includes the modification for a specific amino acid, and by comparing the same with the amino acid sequence database of the protein which is already in possession, the amino acid sequence of the peptide and the modified location may be found. Herein, the peptide fragmentation method may be classified into collision-induced dissociation (CID), electron-capture dissociation (ECD), and electron-transfer dissociation (ETD) depending on the method used. The commonly used fragmentation method is the CID method, which traps peptide ions for fragmentation with an inert gas (argon, helium, nitrogen gas) and then makes the peptide ions excited with strong energy. Then, fragmentation proceeds through collisions between ions. However, fragmentation using the CID method tends to fragment the modified functional group better than the peptide skeleton. This tendency eventually has a weakness in inferring the amino acid sequence of the peptide. The ECD fragmentation method first introduced by McLafferty in 1998 demonstrated that the peptide skeleton is more efficiently fragmented in FT-ICR-MS. ECD cuts the N-Cα skeleton of the peptide through electron energy generated from an electron gun, and as a result, ions of c-/z-type are formed. ETD introduced by Hunt has a tendency to cut peptide skeletons as in ECD, but is a fragmentation method that cuts C-N skeletons and forms ions of b-/y-type.

### Disclosure of the Invention

### Technical Goals

An aspect of the present disclosure provides a protein for neutralizing a coronavirus. Specifically, an aspect thereof presents a novel protein in which virus binding ability is maintained but toxicity is eliminated on the basis of virus binding properties of bean-derived lectins, and thus provides a material for preventing the infection of a coronavirus or alleviating infectious diseases.

### Technical Solutions

According to an aspect, there is provided a composition for neutralizing a coronavirus, wherein the composition includes a protein including amino acids of the following sequence as an active ingredient.

ELDTYPNTDIGDPSYPHIGIDIKSVRSKKTAKWNMQDGKVGTAHIIYNS
VDKRLSAVVSYPNADATSVSYDVDLNDVLPEWVRVGLSASTGLYKETNTILSW
SFTSKLKSNSTHQTDALHFMFNQFSKDQKDLILQGDATTGTDGNLELTRVSSNG
SPEGSSVGRALFYAPVHIWESSATVSAFEATFAFLIK

The protein may be derived from bean-derived protein lectin.

More specifically, the protein may be extracted from fermented Canavalia ensiformis, and an amino acid of a lectin toxic portion may be deleted from ConcanavalinA derived from Canavalia ensiformis.

The amino acid of the lectin toxic portion may include 1^{st} to 7^{th} amino acids and amino acids after a 200^{th} amino acid in ConcanavalinA.

A pharmaceutical composition, a food composition, a health functional food composition, a quasi-drug composition, and a composition for adding feed may be provided by including the composition for neutralizing a coronavirus presented in an example embodiment of the present disclosure as an active ingredient.

The novel protein of an example embodiment of the present disclosure is designed to eliminate toxicity without losing the original lectin property that binds to the virus through a specific fermentation process. An example embodiment of the present disclosure is designed to provide an original patent for a universal virus capture protein composition in the pertinent field, and to provide a commercial benefit of producing a target protein in various ways by revealing the sequence of a protein capable of neutralizing a virus without toxicity.

As used herein, the term "lectin" refers to a carbohydrate-binding protein, and concanavalinA is mannose or glucose-binding lectin, which is one of the major lectins. As used herein, the term "concanavalinA" is used in the same meaning as the term "lectin."

The protein for neutralizing a virus of an example embodiment of the present disclosure has universal utilization as a virus-binding protein.

According to an example embodiment of the present disclosure, the lectin protein is derived from beans, and the beans may be Carnavalia ensiformis, Glycine max, or Canavalia lineata, but is not limited thereto.

According to an example embodiment of the present disclosure, the fermented strain of beans may be one or more fermented strains selected from the group consisting of the genus Lactobacillus, the genus Leuconostoc, the genus Bacillus, genus Weissella, and yeast. More specifically, the fermented strain is one or more fermented strains selected from the group consisting of Lactobacillus brevis (KACC 14481), Lactobacillus buchneri (ATCC 4005), Leuconostoc mesenteroides (KCTC 3505), Bacillus Subtilis, and Bacillus Subtilis Natto.

When the composition of an example embodiment of the present disclosure is prepared into a pharmaceutical composition, the pharmaceutical composition of the present disclosure includes a pharmaceutically acceptable carrier. The pharmaceutically acceptable carrier included in the pharmaceutical composition of an example embodiment of the present disclosure is one commonly used in formulations and includes lactose, dextrose, sucrose, sorbitol, mannitol, starch, gum acacia, calcium phosphate, alginate, gelatin, calcium silicate, microcrystalline cellulose, polyvinylpyrrolidone, cellulose, water, syrup, methyl cellulose, methyl hydroxybenzoate, propyl hydroxybenzoate, talc, magnesium stearate, mineral oil, and the like, but is not limited thereto. The pharmaceutical composition of an example embodiment of the present disclosure may further include, in addition to aforesaid ingredients, a lubricant, a wetting agent, a sweetener, a flavor, an emulsifier, a suspending agent, a preservative, or the like. Suitable pharmaceutically acceptable carriers and formulations are described in detail in Remington's Pharmaceutical Sciences (19th ed., 1995).

The pharmaceutical composition of an example embodiment of the present disclosure may be administered orally or parenterally, and is preferably applied by oral administration.

A suitable dosage of the pharmaceutical composition of an example embodiment of the present disclosure may vary depending on various factors including formulation method, administration method, age, weight, gender or pathological status of a patient, diet, administration time, administration route, excretion rate and response sensitivity. The pharmaceutical composition of an example embodiment of the present disclosure may be generally administered to an adult in an amount of 0.0001 to 100 mg/kg a day.

The pharmaceutical composition of an example embodiment of the present disclosure may be prepared according to a method that may be easily carried out by those skilled in the art in unit-dose forms or in multi-dose packages using a pharmaceutically acceptable carrier and/or excipient. In this connection, the formulation may be a solution in oil or an aqueous medium, a suspension, a syrup, an emulsifying solution form, or a form of an extract, elixirs, powders, granules, a tablet or a capsule, and may further include a dispersing agent or a stabilizing agent.

According to an example embodiment of the present disclosure, the composition for neutralizing the virus is a food composition.

The composition of an example embodiment of the present disclosure may be provided as a food composition. When the antiviral composition of an example embodiment of the present disclosure is prepared into a food composition, the food composition may include, as an active ingredient, a fermentation product of a Canavalia ensiformis crushed body or a Canavalia ensiformis extract, as well as ingredients commonly added during food production, for example, a protein, a carbohydrate, a fat, a nutrient, a seasoning agent, and a flavoring agent. Examples of the aforementioned carbohydrate include a monosaccharide such as glucose, and fructose; a disaccharide such as maltose, sucrose, and oligosaccharide; and a polysaccharide, for example, a common sugar such as dextrin and cyclodextrin, and a sugar alcohol such as xylitol, sorbitol and erythritol. The flavoring agent may include a natural flavoring agent [thaumatin, stevia extract (for example, rebaudioside A, glycyrrhizin, and the like)], and a synthetic flavoring agent (saccharin, aspartame, and the like). For example, in case the food composition of an example embodiment of the present disclosure is made into drinks, citric acid, liquid fructose, a sugar, glucose, acetic acid, malic acid, a fruit juice, eucommia extract, jujube extract, licorice extract, or the like may be added in addition to the fermentation product of the Canavalia ensiformis crushed body or the Canavalia ensiformis extract of an example embodiment of the present disclosure.

The composition for neutralizing the virus of an example embodiment of the present disclosure may be prepared as a health functional food. The health functional food is not particularly limited thereto, but may be any form of food such as health functional food, nutritional supplement, nutraceuticals, pharmafood, health supplement, nutraceutical, designer food, and food additive. Preferable examples thereof include meat, sausage, bread, chocolates, candies, snacks, confectionery, pizza, ramen, other noodles, gums, dairy products such as ice cream, various soups, beverages, tea, drinks, alcohol drinks, vitamin complex, and the like.

The health functional food of an example embodiment of the present disclosure includes ingredients commonly added during food production, for example, a protein, a carbohydrate, a fat, a nutrient, a seasoning agent, and a flavoring agent. Examples of the aforementioned carbohydrate include a monosaccharide such as glucose, and fructose; a disaccharide such as maltose, sucrose, and oligosaccharide; and a polysaccharide, for example, a common sugar such as dextrin and cyclodextrin, and a sugar alcohol such as xylitol, sorbitol and erythritol. The flavoring agent may include a natural flavoring agent [thaumatin, stevia extract (for example, rebaudioside A, glycyrrhizin, and the like)], and a synthetic flavoring agent (saccharin, aspartame, and the like). In addition, the food of an example embodiment of the present disclosure may include various nutrients, vitamins, minerals (electrolytes), dietary ingredients, flavors such as synthetic flavorings and natural flavorings, colorants and enhancers (cheese, chocolate, and the like), pectic acid and salts thereof, alginic acid and salts thereof, organic acids, protective colloid thickeners, pH adjusting agents, stabilizers, preservatives, glycerin, alcohols, and carbonating agents used in carbonated beverages.

According to an example embodiment of the present disclosure, the composition for neutralizing the virus is a composition for adding feed.

The composition for neutralizing virus including the protein of an example embodiment of the present disclosure as an active ingredient may be added to feed to suppress viral infection of animals.

The composition for adding feed of an example embodiment of the present disclosure may further include one or more additives selected from organic acids, such as citric acid, fumaric acid, adipic acid, lactic acid, and malic acid, phosphoric acid salts, such as sodium phosphate, potassium phosphate, dihydrogen pyrophosphate, and polyphosphate, and natural antioxidants, such as polyphenol, catechin, alpha-tocopherol, rosemary extract, vitamin C, green tea extract, licorice extract, chitosan, tannic acid, and phytic acid.

Examples of the composition for adding feed of an example embodiment of the present disclosure may include auxiliary ingredients, such as amino acids, inorganic salts, vitamins, antibiotics, antibacterial substances, antioxidant/antifungal enzymes, active and other viable forms of microbial preparations, and additives such as nutritional supplements, digestion-absorption improvers, growth promoters, or prophylactic agents.

The composition for adding feed of an example embodiment of the present disclosure may be in the form of a dry or liquid preparation and may further include an excipient for adding feed. The excipient for adding feed may be, for example, zeolite, corn flour, rice bran, and the like, without being limited thereto.

The composition for adding livestock feed of an example embodiment of the present disclosure may be administered to animals alone or in combination with other feed additives included in an edible carrier. In addition, a daily dosage may be employed via once-daily dose or multiple-divided daily dose as commonly known in the art to which the present disclosure pertains.

Examples of animals to which the composition for adding feed of an example embodiment of the present disclosure is applied may include livestock such as beef cattle, dairy cattle, calves, pigs, piglets, sheep, goats, horses, rabbits, dogs, and cats; and poultry such as chicks, hens, domestic chickens, roosters, ducks, geese, turkeys, quails, and small birds, without being limited thereto.

### Effects

A composition for neutralizing a virus according to an example embodiment of the present disclosure may be used as a pharmaceutical composition for preventing or treating the infection of a coronavirus, a protein medicine for respiratory treatment for preventing the infection of a coronavirus or alleviating infectious diseases, a food composition, a quasi-drug composition, or a composition for adding feed.

### Brief Description of Drawings

FIG. 1 shows the results of isolation of proteins obtained from fermented Canavalia ensiformis using LC.
FIG. 2 shows the results of mass value information for peptides identified from fermented Canavalia ensiformis.
FIG. 3 shows the results of ETD amino acid sequencing of the protein as an active substance.
FIG. 4 shows the mass spectrometry spectrum and deconvolution results for tConA.

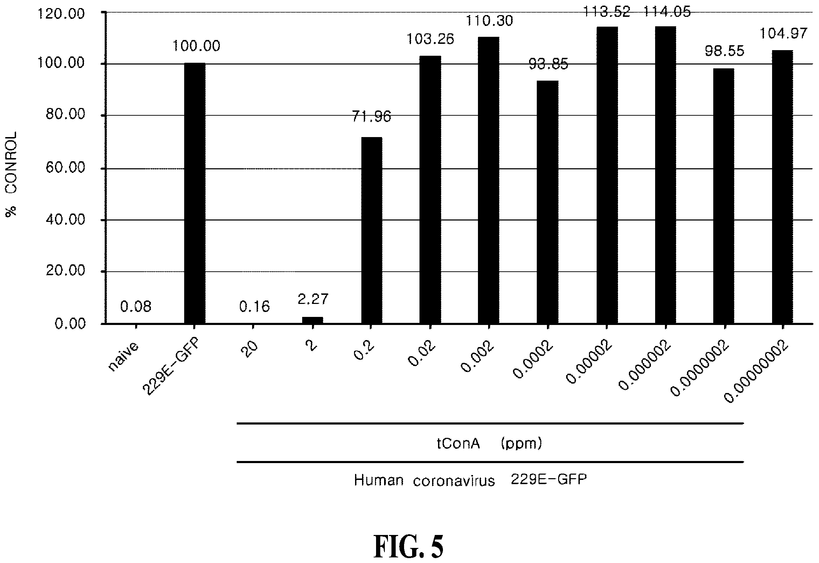

FIG. 5 is a graph showing the human coronavirus neutralizing ability of tConA.

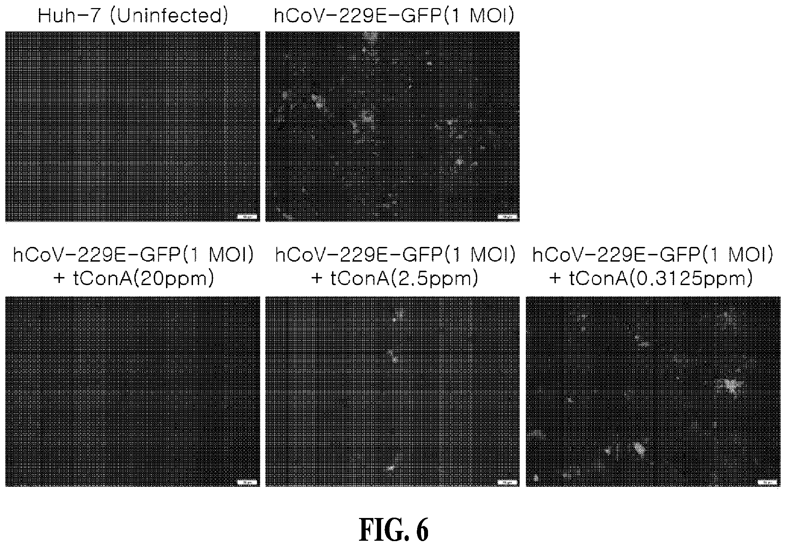

FIG. 6 shows a verification image of the human coronavirus neutralizing ability of tConA.

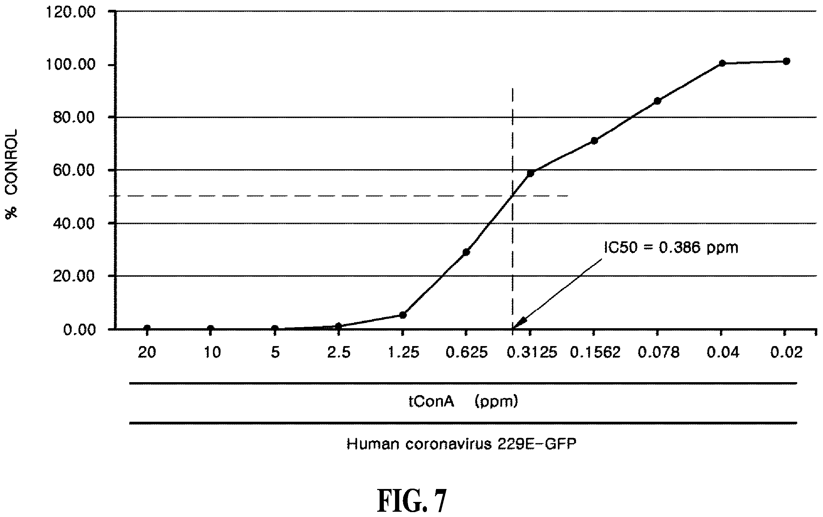

FIG. 7 shows the IC50 values of tConA for neutralizing a coronavirus.

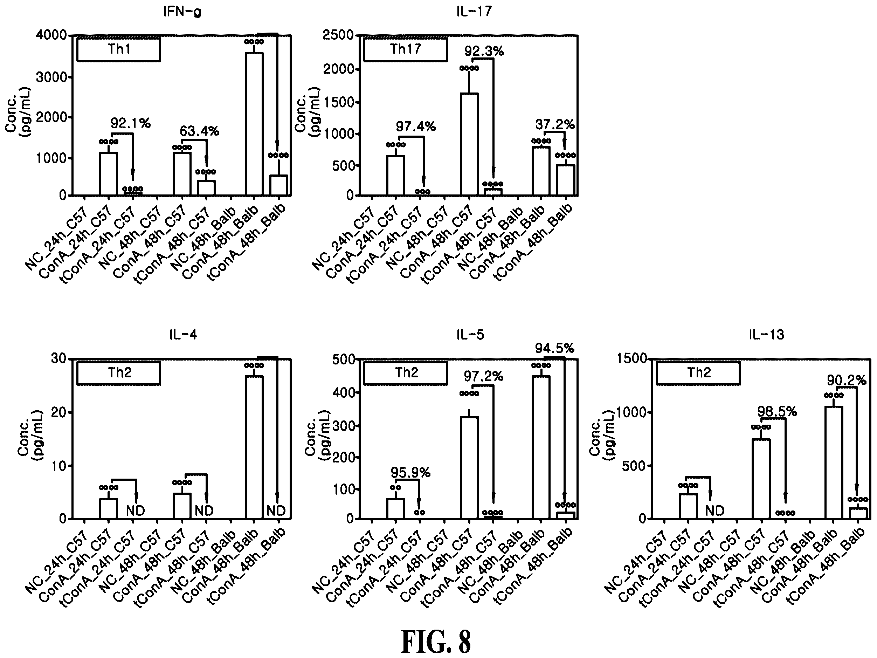

FIG. 8 is a comparison of the cytokine production ability of splenocytes of ConA and tConA.

### Best Mode for Carrying Out the Invention

### Example 1. Obtaining Raw Materials through Fermenting Canavalia Ensiformis

200 ml (1% of tryptone, 0.5% of yeast extract, and 1% of sodium chloride) of LB broth miller (LBL407.500, Bioshop, Canada) containing tryptone, yeast extract and sodium chloride was additionally added with 8.25 g of Canavalia ensiformis powder, inoculated with a strain corresponding to 5% (v/v) of a medium, and then fermented for 7 days by stirring at 33°C. The fermented supernatant was centrifuged at 12,000 rpm, 4°C for 20 minutes, the supernatant was collected, the residue was fractionated using filter paper (Whatman filter paper 4, 25 nm), and the filtrate was lyophilized by removing salts using a 10 kDa membrane filter (Sartorius 7554-95, MASTERFLEX, USA) and concentrating the fermented liquid capacity by 2.4 times. The obtained solid content was the final product of the fermented Canavalia ensiformis, and SDS-PAGE was performed to check the fermentation termination time.

From day 0 to day 7 of fermentation, 1 mg of each sample of the fermented Canavalia ensiformis was taken and dissolved in 1 ml of distilled water and subjected to SDS-PAGE analysis through 15% of polyacrylamide gel. A major band was observed at 48 kDa molecular weights on day 0 of fermentation. However, from day 1 of fermentation, the band with a molecular weight of 48 kDa was decreased, and the band of 25 kDa or less, the size of ConA, was increased. By day 7, a major band was observed more clearly at molecular weights of 25 kDa or less.

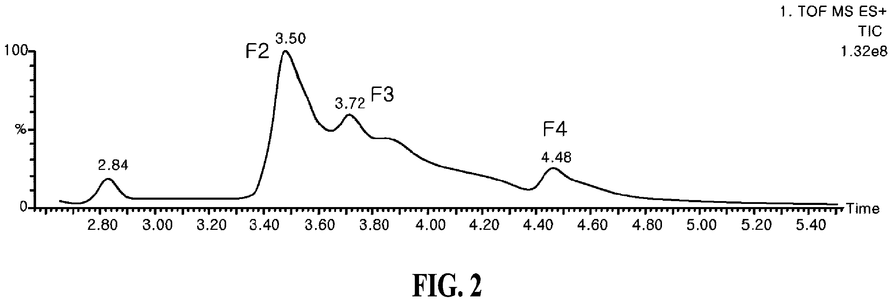

As a result of isolation by size using 15% SDS-PAGE, a protein of about 48 kDa exists as the main protein on day 0 of fermentation, and three types of protein of about 20 to 25 kDa were produced when fermented for 4 days using Bacillus subtillis natto. Through peptide coverage analysis, specific homology was identified from the standard material, ConA, and the fermented Canavalia ensiformis. After cutting the F0 band of Canavalia ensiformis powder (column 2 in FIG. 2) and the F2, F3 and F4 bands of fermented Canavalia ensiformis (column 3 in FIG. 2), in-gel digestion was performed to wash with 25 mM ABC in 50% ACN solution. After decomposition into peptides with trypsin gold (Promega, V5280) under 50 mM ABC conditions, desalting was performed using Oasis SPE (Waters, 186001828BA), and peptide analysis was performed using nanoUPLC-Synapt G2 si (Waters, USA) equipment. The results obtained through LC-MS were analyzed using PLGS (ProteinLynx Global Server (version 3.0, PLGS, Waters). As a result, in the case of F0 band, CVJB concanavalinA Jack bean (UniProtKB: CVJB Con A, gi72333) expected to be a commercial ConA sequence and 97.5% of homology were identified, and in the case of F2, F3, and F4 bands, CVJB concanavalinA, Jack bean (UniProtKB: CVJB Con A, gi72333) and each sequence coverage were identified as 81.4%, 53.2%, and 62.86%.

**[Table 1]**

| Band name | Access number | Description | Score | Same peptide | Sequence cover (%) |
|---|---|---|---|---|---|
| F0 | gi72333 | CVJB concanavalinA jack bean | 2389.3 | 23 | 97.5 |
| F2 | gi72333 | CVJB concanavalinA jack bean | 730.1 | 9 | 81.4 |
| F3 | gi72333 | CVJB concanavalinA jack bean | 629.7 | 8 | 53.2 |
| F4 | gi72333 | CVJB concanavalinA jack bean | 1341.8 | 8 | 62.86 |

### Example 2. Active Ingredient Identification

In order to measure the exact molecular weight of the proteins isolated from the fermented Canavalia ensiformis, the intact protein mass values were calculated for the main band on the day 0 of fermentation of Canavalia ensiformis and the protein concentrate with a molecular weight of 30 KDa or less, which was identified in the fermented product on day 4 of fermentation of Canavalia ensiformis. Molecular weight measurement was performed using nano ultra-high performance liquid chromatography (UPLC, ACQUITY UPLC I-Class/SYNAPT G2-S HDMS, Waters) for fermentation analysis, and the analysis conditions are as shown below.

An ACQUITY BEH300 C18 (1.7 µm × 2.1 × 50 mm) column was used, a column temperature at 60°C, mobile phase solvent A (0.1% of formic acid in water), and solvent B (0.1% of formic acid in acetonitrile) were used. The mass spectrometer performed analysis in the ESI positive mode, and the analysis conditions were set to a capillary voltage of 3.0 kV, a cone voltage of 30 V, a source temperature of 120°C, and a scan time of 0.5 seconds. ESI prot 1.0 was calculated and the deconvoluted MW (Da) and std values as shown in Table 2 were set.

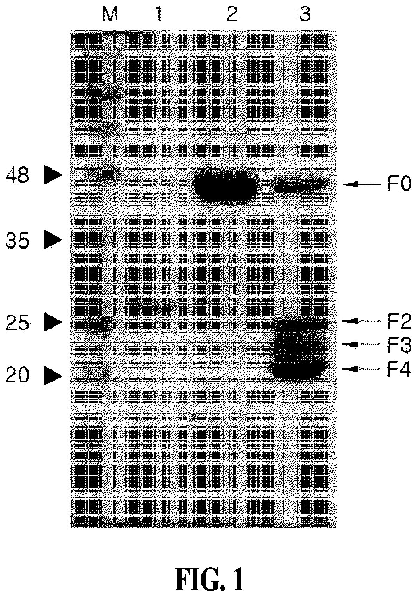

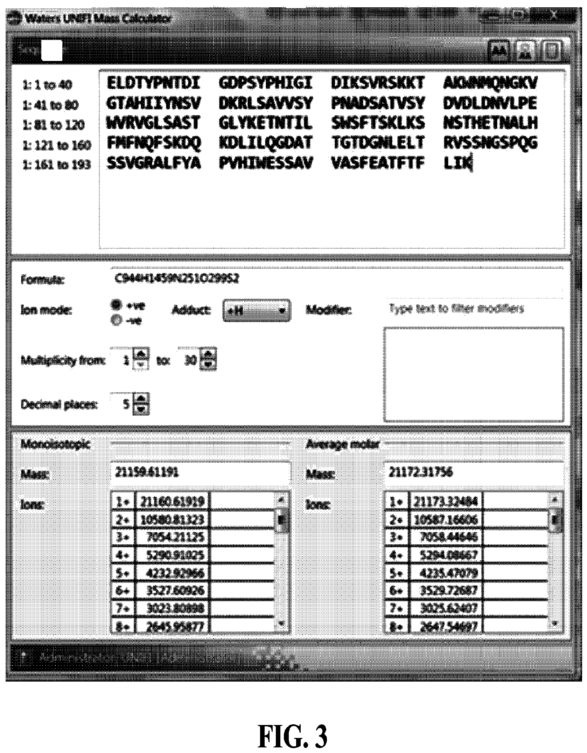

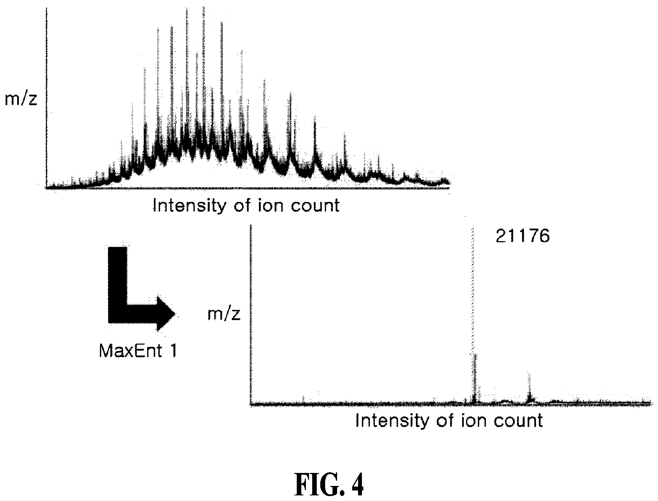

For the F2, F3 and F4 bands, as CVJB concanavalinA, Jack bean (UniProtKB: CVJB Con A, gi72333) and each sequence coverage were identified as 81.4%, 53.2%, and 62.86%, due to the nature of trypsin, which recognizes lysine and arginine residues, the exact cleavage position may not be checked. However, F2 was predicted to be the protein (26213.96 Da) of the F2 region of FIG. 4 identified when the mass value of the intact protein was measured with ConA in a monomer state. As a result of calculating the mass value of the F3 band, 21175.48 Da was predicted to be the protein (21176 Da) of the F3 region of FIG. 3 identified when the mass value of the intact protein was measured. In the case of the F4 band, as the mass value was calculated as 18796.86 Da, it was predicted to be the protein (18796.8 Da) of the F4 region of FIG. 3 identified when the mass value of the intact protein was measured (FIG. 1 and Table 2).

**[Table 2]**

| | Charge (+) | Peak (m/z) | MW(Da) | Deconvoluted MW (Da) |
|---|---|---|---|---|
| F2 | 29 | 904.9487 | 26214.2820 | 26213.9637 ± 0.7529 |
| | 28 | 937.2229 | 26214.0188 | |
| | 27 | 971.8983 | 26214.0397 | |
| | 26 | 1009.2819 | 26215.1229 | |
| | 25 | 1049.5328 | 26213.1215 | |
| | 24 | 1093.2675 | 26214.2294 | |
| | 23 | 1140.7080 | 26213.1019 | |
| | 22 | 1192.5895 | 26214.7943 | |
| | 21 | 1249.2468 | 26213.0160 | |
| | 20 | 1311.7194 | 26214.2292 | |
| | 19 | 1380.5923 | 26212.1028 | |
| | 18 | 1457.2847 | 26212.9816 | |
| | 17 | 1543.1046 | 26215.64332 | |
| F3 | 28 | 757.2388 | 21174.4640 | 21175.4820 ± 0.9101 |
| | 27 | 785.3299 | 21176.6929 | |
| | 26 | 815.4147 | 21174.5783 | |
| | 25 | 848.0338 | 21175.6465 | |
| | 24 | 883.3274 | 21175.6670 | |
| | 23 | 921.6443 | 21174.6362 | |
| | 22 | 963.4954 | 21174.7241 | |
| | 21 | 1009.4067 | 21176.3739 | |
| | 20 | 1059.8358 | 21176.5572 | |
| | 19 | 1121.5699 | 21290.6772 | |
| | 18 | 1177.6122 | 21178.8766 | |
| | 17 | 1246.6261 | 21175.5087 | |

### Example 3. Sequence Analysis of Fermented Canavalia Ensiformis

The protein sequence of the fermented Canavalia ensiformis obtained above was analyzed. ETD was used for F3 among fermentation proteins. As a result of identifying the amino acid sequence, it was found to be a novel protein in which some amino acids were deleted compared to the known protein ConcanavalinA. From these results, it was assumed that some of the amino acids were cleaved during the fermentation process, and the novel protein was named truncated ConcanavalinA (tConA).

**[Table 3]**

| tConA | ConA |
|---|---|
| ELDTYPNTDIGDPSYPHIGIDIKSV RSKKTAKWNMQDGKVGTAHIIY NSVDKRLSAVVSYPNADATSVSY DVDLNDVLPEWVRVGLSASTGLY KETNTILSWSFTSKLKSNSTHQTD ALHFMFNQFSKDQKDLILQGDAT TGTDGNLELTRVSSNGSPEGSSVG RALFYAPVHIWESSATVSAFEATF AFLIK | ADTIVAVELDTYPNTDIGDPSYPHI GIDIKSVRSKKTAKWNMQDGKVG TAHIIYNSVDKRLSAVVSYPNADA TSVSYDVDLNDVLPEWVRVGLSA STGLYKETNTILSWSFTSKLKSNST HQTDALHFMFNQFSKDQKDLILQ GDATTGTDGNLELTRVSSNGSPEG SSVGRALFYAPVHIWESSATVSAF EATFAFLIKSPDSHPADGIAFFISNI DSSIPSGSTGRLLGLFPDAN |
| Theoretical pI/Mw: 5.47 / 21133.55 | Theoretical pI/Mw: 5.00 / 25572.38 Da |

### Example 4. Identification of Human Coronavirus Neutralizing Power of tConA

The virus to be identified was Human Coronavirus, which was treated with 1X 10^5 GFP-transducing units/200 µl and was used. When applied to the experiment, it was applied by diluting 2 to 10 times in DMEM media in a unit of 100 ppm (50 µl). The treatment time is 1 hour and the treatment temperature is 4°C.

Virus inoculation was performed on huh-7 cells (human hepatocellular carcinoma cell line), and cells were prepared as 1 × 10^5 cells/well in a 24-well plate. After washing the culture supernatant, 250 µl of the virus-tConA mixture was inoculated into Huh-7 cells. The unbound virus was washed 3 times in DEME medium supplemented with 2% FBS and 1X penicillin/streptomycin. After culturing the washed cells for 24 hours, FACS analysis was performed in DEME medium supplemented with 2% FBS and 1X penicillin/streptomycin.

At MOI = 1, the percentage of infected cells is theoretically 37% based on the Poisson distribution. The percentage of infection with untreated virus ranged from 31% to 41% in repeated experiments, with a mean of 37%. Data for the untreated virus samples indicate that the testing process performed as expected. The percentage of GFP-positive cells in the virus-only sample was normalized to 100% for comparison of each independent test.

tConA was diluted 10-fold and mixed with the virus to identify the virus neutralizing ability of tConA. Inhibition of viral entry was identified up to 1.25 ppm. At 0.2 ppm, the inhibitory effect was lower than 30%, indicating that the IC50 would be between 1.25 ppm and 0.2 ppm.

In order to determine the IC50 of tConA against virus, tConA was serially diluted 2-fold and mixed with virus. Up to 2.5 ppm, the inhibitory effect was 99% or higher. The IC50 of tConA is estimated to be 0.386 ppm.

In conclusion, tConA was 99% or higher effective in inhibiting viral entry into Huh-7 cells down to 1.25 ppm. The IC50 is estimated to be 0.386 ppm.

### Example 5. Identification of Toxicity Reducing Effect of tConA

After killing two normal mouse species (C57BL/6, Balb/C) with ether, the spleen was aseptically extracted to prepare a spleen cell liquid, and then 500 µl (4 × 10⁶ cells/ml) of the cell liquid was added to a 24 well flat-bottomed plate. After stabilization by culturing for 30 minutes (37°C, 5% CO2), 100 µl (2 µg/ml) of ConA and tConA and 300 µl of 10% FBS-RPMI medium were added to each well, and the culture solution obtained by culturing for 24 hours and 48 hours was used while being stored at -70°C. According to the sandwich ELISA method provided by PharMingen, the amount of cytokine (IFN-γ, IL-17, IL-4, IL-5, IL-13) production in the culture medium was compared and measured. In the case of C57BL/6, when only 48 hours results were compared, IFN-γ was reduced by 63.4% compared to ConA when treated with tConA, IL-17 was reduced by 92.3%, IL-4 was not detected, and IL-5 was reduced by 97.2%. As such, tConA maintains its antiviral ability and lowers its toxicity, so it is expected to have a wide range of applications when applied to humans or animals.

### Sequence List Fee Text

ELDTYPNTDIGDPSYPHIGIDIKSVRSKKTAKWNMQDGKVGTAHIIYNS
VDKRLSAVVSYPNADATSVSYDVDLNDVLPEWVRVGLSASTGLYKETNTILSW
SFTSKLKSNSTHQTDALHFMFNQFSKDQKDLILQGDATTGTDGNLELTRVSSNG
SPEGSSVGRALFYAPVHIWESSATVSAFEATFAFLIK

## Claims

1. A composition for neutralizing a coronavirus, wherein the composition comprises a protein comprising amino acids of the following sequence as an active ingredient.

ELDTYPNTDIGDPSYPHIGIDIKSVRSKKTAKWNMQDGKVGTAHIIYNS
VDKRLSAVVSYPNADATSVSYDVDLNDVLPEWVRVGLSASTGLYKETNTILSW
SFTSKLKSNSTHQTDALHFMFNQFSKDQKDLILQGDATTGTDGNLELTRVSSNG
SPEGSSVGRALFYAPVHIWESSATVSAFEATFAFLIK

2. The composition of claim 1, wherein the protein is derived from bean-derived lectin protein.

3. The composition of claim 1, wherein the protein is extracted from fermented Canavalia ensiformis.

4. The composition of claim 1, wherein, for the protein, an amino acid of a lectin toxic portion is deleted from ConcanavalinA derived from Canavalia ensiformis.

5. The composition of claim 4, wherein the amino acid of the lectin toxic portion comprises 1^{st} to 7^{th} amino acids and amino acids after a 200^{th} amino acid in ConcanavalinA.

6. A pharmaceutical composition comprising the composition for neutralizing a coronavirus of claim 1 as an active ingredient.

7. A food composition comprising the composition for neutralizing a coronavirus of claim 1 as an active ingredient.

8. A health functional food composition comprising the composition for neutralizing a coronavirus of claim 1 as an active ingredient.

9. A quasi-drug composition comprising the composition for neutralizing a coronavirus of claim 1 as an active ingredient.

10. A composition for adding feed, the composition comprising the composition for neutralizing a coronavirus of claim 1 as an active ingredient.
